# EUROPÄISCHE PATENTSCHRIFT

(11) **EP 3 827 731 B1**
(45) Veröffentlichungstag und Bekanntmachung des Hinweises auf die Patenterteilung: **26.03.2025**
(21) Anmeldenummer: 20209721.8
(22) Anmeldetag: 25.11.2020
(51) Int. Cl.: A61B 1/00, A61B 1/005, A61B 1/012, A61B 1/05

(54) **ENDOSKOPVORRICHTUNG FÜR EIN FLEXIBLES ENDOSKOP UND VERFAHREN ZU DEREN HERSTELLUNG**
ENDOSCOPE DEVICE FOR FLEXIBLE ENDOSCOPE AND METHOD OF MANUFACTURE THEREOF
DISPOSITIF POUR ENDOSCOPE FLEXIBLE ET METHODE DE FABRICATION CORRESPONDANTE

(30) Priorität: 26.11.2019 US 201962940650 P
(43) Veröffentlichungstag der Anmeldung: 02.06.2021
(73) Patentinhaber: Karl Storz SE & Co. KG, 78532 Tuttlingen (DE)
(72) Erfinder: POLLUKS, Indrek-Toomas, 78532 Tuttlingen (DE); FITZPATRICK, Craig, 78532 Tuttlingen (DE)

(56) Entgegenhaltungen:
- DE-A1- 10 122 203
- US-A- 5 789 047
- US-A1- 2003 220 545
- US-A1- 2004 106 853
- US-A1- 2009 112 066
- US-A1- 2010 075 075
- US-A1- 2010 256 445

## Beschreibung

### Stand der Technik

Die Erfindung betrifft eine Endoskopvorrichtung gemäß dem Oberbegriff des Anspruchs 1 sowie ein Verfahren zur Herstellung einer Endoskopvorrichtung gemäß dem Oberbegriff des Anspruchs 18.

Es sind bereits Endoskopvorrichtungen bekannt, welche wenigstens einen Arbeitskanal aufweisen, welcher einen schlauchförmigen Arbeitskanalmantel aufweist, welcher ein Arbeitskanalvolumen des Arbeitskanals begrenzt, in welches beispielsweise wenigstens ein chirurgisches Instrument anordenbar ist, wobei der Arbeitskanalmantel wenigstens eine Mantelschicht besitzt, welche wenigstens ein Kunststoffmaterial umfasst, und wenigstens eine weitere Mantelschicht besitzt, welche wenigstens ein weiteres Kunststoffmaterial umfasst, welches von dem Kunststoffmaterial der Mantelschicht verschieden ausgebildet ist. Derartige Endoskopvorrichtungen sind beispielsweise in den Dokumenten US 2003/220545 A1, US 2009/112066 A1, US2010/075075 A1, DE 101 22 203 A1, US 2010/256455 A1 und US 2004/106853 A1 beschrieben,
Die Aufgabe der Erfindung besteht insbesondere darin, eine gattungsgemäße Vorrichtung mit verbesserten Eigenschaften eines Arbeitskanals bereitzustellen, wobei der Arbeitskanal vorteilhaft flexibel ausgestaltet ist. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Endoskopvorrichtung für ein Endoskop, insbesondere ein flexibles Endoskop, mit wenigstens einem Arbeitskanal, welcher einen schlauchförmigen Arbeitskanalmantel aufweist, welcher ein Arbeitskanalvolumen des Arbeitskanals begrenzt, wobei der Arbeitskanalmantel wenigstens eine Mantelschicht aufweist, welche wenigstens ein Kunststoffmaterial umfasst, und wenigstens eine weitere Mantelschicht aufweist, welche wenigstens ein weiteres Kunststoffmaterial umfasst, welches von dem Kunststoffmaterial der einen Mantelschicht verschieden ist.

Es wird vorgeschlagen, dass die Mantelschicht entlang der Haupterstreckung des Arbeitskanals eine konstante Schichtdicke aufweist und dass die weitere Mantelschicht entlang der Haupterstreckung des Arbeitskanals abschnittsweise eine variable Schichtdicke aufweist. Durch Variation der Schichtdicke kann eine Anordnung des Arbeitskanals, insbesondere innerhalb eines Endoskopschafts und/oder an einer Endoskopspitze verbessert werden. Weiter vorteilhaft kann eine Stabilität des Arbeitskanals verbessert werden.

Unter einer "Endoskopvorrichtung" soll insbesondere ein, vorzugsweise funktionsfähiger, Bestandteil eines Endoskops, insbesondere eine Unterbaugruppe und/oder eine Konstruktions- und/oder eine Funktionskomponente eines Endoskops, verstanden werden. Alternativ kann die Endoskopvorrichtung ein Endoskop zumindest teilweise, vorzugsweise zumindest zu einem Großteil und besonders bevorzugt vollständig ausbilden. Unter dem Ausdruck "zumindest zu einem Großteil" soll dabei insbesondere zumindest zu 55 %, vorzugsweise zumindest zu 65 %, bevorzugt zumindest zu 75 %, besonders bevorzugt zumindest zu 85 % und ganz besonders bevorzugt zumindest zu 95 %, sowie vorteilhaft vollständig verstanden werden und zwar insbesondere mit Bezug auf ein Volumen und/oder eine Masse eines Objekts. Die Endoskopvorrichtung ist beispielsweise dazu eingerichtet, zumindest teilweise und vorzugsweise zumindest zu einem Großteil in eine künstliche oder natürliche Öffnung, insbesondere Körperöffnung, eingeführt zu werden, um dort eine Behandlung oder Untersuchung vorzunehmen. Unter "eingerichtet" soll insbesondere speziell programmiert, vorgesehen, ausgelegt, ausgebildet und/oder ausgestattet verstanden werden. Darunter, dass ein Objekt zu einer bestimmten Funktion eingerichtet ist, soll insbesondere verstanden werden, dass das Objekt diese bestimmte Funktion in zumindest einem Anwendungs- oder Betriebszustand erfüllt oder ausführt.

Die Endoskopvorrichtung weist insbesondere einen Endoskopschaft auf, innerhalb welchem der Arbeitskanal angeordnet ist. Der Endoskopschaft ist insbesondere dazu eingerichtet, wenigstens teilweise und vorzugsweise wenigstens zu einem Großteil in eine insbesondere künstliche oder natürliche Öffnung, insbesondere Körperöffnung, eingeführt zu werden. Unter "distal" soll insbesondere bei einer Bedienung einem Patienten zugewandt und einem Bediener abgewandt verstanden werden. Unter "proximal" soll insbesondere bei einer Bedienung einem Patienten abgewandt und einem Bediener zugewandt verstanden werden. Der Arbeitskanal ist als ein längliches Objekt ausgebildet und beispielsweise dazu geeignet, darin ein chirurgisches Instrument anzuordnen oder um darin Flüssigkeiten oder Gase zu transportieren. Unter einem "länglichen Bauteil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine Erstreckung des Bauteils zumindest im Wesentlichen senkrecht zur Haupterstreckung, also insbesondere einem Durchmesser des Bauteils. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung und die Bezugsrichtung einen Winkel von 0° insbesondere unter Berücksichtigung einer maximalen Abweichung von kleiner als 8°, vorteilhaft von kleiner als 5° und besonders vorteilhaft von kleiner als 2° einschließt. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90° insbesondere unter Berücksichtigung einer maximalen Abweichung von kleiner als 8°, vorteilhaft von kleiner als 5° und besonders vorteilhaft von kleiner als 2° einschließt. Unter einer "Haupterstreckung" eines Objekts soll dabei insbesondere eine Erstreckung des Objekts verstanden werden, welche entlang einer längsten Kante eines kleinsten gedachten Quaders verläuft, welcher das Objekt gerade noch vollständig umschließt.

Der Arbeitskanalmantel weist insbesondere einen Innendurchmesser auf, welcher wenigstens 0,5 mm, vorzugsweise wenigstens 1,5 mm und besonders bevorzugt wenigstens 2 mm und/oder höchstens 12 mm, vorzugsweise höchstens 5 mm und besonders bevorzugt höchstens 3 mm beträgt. Die Mantelschicht weist insbesondere eine Schichtdicke auf, welche wenigstens 1,5 %, vorzugsweise wenigstens 2 % und besonders bevorzugt wenigstens 3 % und/oder höchstens 10 %, vorzugsweise höchstens 7 % und besonders bevorzugt höchstens 4 % des Innendurchmessers beträgt.

Da eine Schichtdicke der weiteren Mantelschicht abschnittsweise entlang der Haupterstreckung des Arbeitskanals variabel ist, weist die weitere Mantelschicht in einem Abschnitt insbesondere eine Schichtdicke auf, welche wenigstens 5 %, vorzugsweise wenigstens 6 % und besonders bevorzugt wenigstens 10 % und/oder höchstens 15 %, vorzugsweise höchstens 13 % und besonders bevorzugt höchstens 11 % des Innendurchmessers entspricht.

Die weitere Mantelschicht kann in dem weiteren Abschnitt des Arbeitskanalmantels eine Schichtdicke aufweisen, die gegenüber der Schichtdicke in dem ersten Abschnitt um mindestens 10%, bevorzugt wenigstens 25%, besonders bevorzugt wenigstens 40% oder mehr als 50% reduziert ist.

Alternativ oder zusätzlich kann die weitere Mantelschicht in einem weiteren Abschnitt der Arbeitskanalmantels kann die weitere Mantelschicht eine Schichtdicke von wenigstens 0,1 % vorzugswiese wenigstens 0,14 % und besonders bevorzugt wenigstens 0,18 % und/oder höchstens 0,28 %, vorzugsweise höchstens 0,24 % und besonders bevorzugt höchstens 0,2 % des Innendurchmessers aufweisen. Der Bauraum im distalen Endabschnitt von Endoskopen, der verschiedene weitere Komponenten wie Bildsensoren, Beleuchtungseinheiten oder Teile des Endoskopschafts aufnimmt, kann so begrenzt sein, dass eine sehr starke Verringerung der Dicke der weiteren Mantelschicht in einigen Abschnitten des Arbeitskanalmantels sinnvoll ist, um weiteren Raum zu schaffen.

Der Arbeitskanal ist insbesondere flexibel ausgebildet. Die Mantelschicht und die weitere Mantelschicht erstrecken sich insbesondere zumindest im Wesentlichen parallel entlang der Haupterstreckung. Die Mantelschicht und die weitere Mantelschicht sind insbesondere koaxial angeordnet. Die weitere Mantelschicht umgibt die Mantelschicht insbesondere entlang eines Umfangs des Arbeitskanalmantels, wenigstens teilweise, vorzugsweise zumindest zu einem Großteil und besonders bevorzugt vollständig. Die Mantelschicht und die weitere Mantelschicht bilden den Arbeitskanalmantel wenigstens teilweise, vorzugsweise wenigstens zu einem Großteil und besonders bevorzugt vollständig aus. Das eine Kunststoffmaterial bildet insbesondere zumindest teilweise, vorzugsweise zumindest zu einem Großteil und besonders bevorzugt vollständig die Mantelschicht aus. In anderen Worten kann die eine Mantelschicht frei von einem weiteren Kunststoff sein. Ferner ist insbesondere die weitere Mantelschicht insbesondere zumindest teilweise, vorzugsweise zumindest zu einem Großteil und besonders bevorzugt vollständig aus dem weiteren Kunststoffmaterial ausgebildet. In anderen Worten kann die weitere Mantelschicht frei von einem zusätzlichen Kunststoff sein. Insbesondere da das eine Kunststoffmaterial und das weitere Kunststoffmaterial voneinander verschieden ausgebildet sind, bilden diese gemeinsam wenigstens teilweise einen Verbundmaterial aus, aus welchem der Arbeitskanalmantel wenigstens teilweise ausgebildet ist. Vorzugsweise sind das Kunststoffmaterial und das weitere Kunststoffmaterial stoffschlüssig und/oder formschlüssig miteinander verbunden.

Durch die Ausbildung der Mantelschichten aus den Kunststoffmaterialien wie zuvor beschrieben kann insbesondere ein Arbeitskanal mit besonders kleinem Durchmesser und geringer Wandstärke geschaffen werden, der sich für Endoskopvorrichtungen und Endoskope mit sehr geringem Durchmesser eignet.

Um den Durchmesser weiter zu verringern, wird vorgeschlagen, dass die Schichtdicke der weiteren Mantelschicht abschnittsweise entlang der Haupterstreckung des Arbeitskanals abnimmt. Vorzugsweise nimmt die Schichtdicke der weiteren Mantelschicht abschnittsweise entlang der Haupterstreckung des Arbeitskanals in distaler Richtung ab. Insbesondere ist ein erster Abschnitt des Arbeitskanalmantels ein proximaler Abschnitt und ein weiterer Abschnitt des Arbeitskanalmantels ein distaler Abschnitt. Ferner kann der Arbeitskanalmantel einen Zwischenabschnitt aufweisen, welcher zwischen dem ersten Abschnitt und dem weiteren Abschnitt angeordnet ist. Vorzugsweise geht die insbesondere größere Schichtdicke des ersten Abschnitts innerhalb des Zwischenabschnitts kontinuierlich in die, insbesondere geringere, Schichtdicke des weiteren Abschnitts über. Der Zwischenabschnitt kann eine Länge von einigen Millimetern, vorzugsweise weniger als 5 mm aufweisen. Die Mantelschicht kann in dem ersten und dem weiteren Abschnitt eine Schichtdicke wie oben beschrieben aufweisen.

Es wird ferner vorgeschlagen, dass der Arbeitskanalmantel wenigstens abschnittsweise frei von der weiteren Mantelschicht ist. Es kann vorteilhaft eine besonders kompakte Ausgestaltung der Arbeitskanalmantels erzielt werden. Beispielsweise könnte der weitere Abschnitt von der zweiten Mantelschicht frei sein. In anderen Worten könnte in dem weiteren Abschnitt eine Schichtdicke der weiteren Mantelschicht den Wert Null besitzen.

Bei dem distalen Abschnitt kann es sich insbesondere um einen distalen Endabschnitt des Arbeitskanalmantels handeln. Bevorzugt ist dieser distale Endabschnitt frei von der weiteren Mantelschicht. Es kann vorteilhaft eine Montage weiter vereinfacht und ein Durchmesser des Arbeitskanals insbesondere im distalen Bereich verkleinert werden.

Die Mantelschicht und die weitere Mantelschicht können zueinander benachbart angeordnet sein. Ferner wird vorgeschlagen, dass die Mantelschicht und die weitere Mantelschicht aneinander anliegen. Hierdurch kann vorteilhaft ein besonders stabiler Arbeitskanalmantel geschaffen werden.

Es wird vorgeschlagen, dass die Mantelschicht und die weitere Mantelschicht stoffschlüssig und/oder formschlüssig miteinander verbunden sind. Es kann vorteilhaft ein Verbundmaterial aus dem einen und dem weiteren Kunststoffmaterial der einen und der weiteren Mantelschicht gebildet werden, beispielsweise unter Anwendung von Druck und Hitze. Dadurch kann eine Delamination, also eine Trennung der beiden Schichten voneinander bei Biegung des Arbeitskanals verhindert werden. Arbeitskanäle in flexiblen Endoskopen sind häufig Biegungen mit kleinem Biegeradius ausgesetzt.

Weiter wird vorgeschlagen, dass die Mantelschicht eine Innenseite des Arbeitskanalmantels ausbildet und die weitere Mantelschicht eine Außenseite des Arbeitskanalmantels ausbildet. Es kann vorteilhaft eine homogene Innenseite bereitgestellt werden, welche von einer etwaigen Ausgestaltung einer die Mantelschicht stabilisierenden weiteren Mantelschicht kaum beeinträchtigt wird.

Es wird ferner vorgeschlagen, dass ein Innendurchmesser des Arbeitskanalmantels entlang der Haupterstreckung des Arbeitskanals konstant oder zumindest im Wesentlichen konstant ist. Es kann vorteilhaft sichergestellt werden, dass ein in dem von dem Arbeitskanalmantel begrenzten Arbeitskanalvolumen angeordnetes chirurgisches Instrument entlang der gesamten Haupterstreckung des Arbeitskanals angeordnet und/oder durch diesen hindurchgeführt werden kann, ohne dass dieses beschädigt wird oder in diesem stecken bleibt. Der Arbeitskanal kann dessen ungeachtet Biegungen oder Abzweigungen aufweisen.

Weiter wird vorgeschlagen, dass ein Außendurchmesser des Arbeitskanalmantels entlang der Haupterstreckung des Arbeitskanals variabel ist. Hierdurch kann insbesondere eine individuelle Anpassung des Arbeitskanalmantels an einen Bauraum innerhalb eines Endoskopschafts erfolgen. In dem einen Abschnitt entspricht der Außendurchmesser insbesondere wenigstens 110 %, vorzugsweise wenigstens 115 %, und besonders bevorzugt wenigstens 120 % und/oder höchstens 145 %, vorzugsweise höchstens 140 % und besonders bevorzugt höchstens 135 % des Innendurchmessers. In dem weiteren Abschnitt der Arbeitskanalmantels weist dieser einen Außendurchmesser von wenigstens 101 % vorzugswiese wenigstens 106 % und besonders bevorzugt wenigstens 110 % und/oder höchstens 138 %, vorzugsweise höchstens 133 % und besonders bevorzugt höchstens 120 % des Innendurchmessers entspricht.

Es wird weiterhin vorgeschlagen, dass wenigstens eines der Kunststoffmaterialien Polytetrafluorethylen (PTFE) ist. Dadurch kann vorteilhaft eine Stabilität verbessert werden, wobei vorzugsweise gleichzeitig eine entsprechende Flexibilität des Arbeitskanalmantels erzielt werden kann. Insbesondere kann der Kunststoff der einen Mantelschicht Polytetrafluorethylen sein.

Alternativ oder zusätzlich kann eines der Kunststoffmaterialien Polyetheretherketon (PEEK) oder ein anderer thermoplastischer Kunststoff sein oder umfassen.

Ferner wird vorgeschlagen, dass das eine Kunststoffmaterial der einen Mantelschicht PTFE ist. Ferner umfasst oder ist der weitere Kunststoff der weiteren Mantelschicht Polytetrafluorethylen, ist jedoch dahingehend verschieden von dem einen Kunststoff ausgebildet, dass er expandiert ist (expandiertes Polytetrafluorethylen, ePTFE). PTFE ist reaktionsträge und weist einen geringen Reibungskoeffizienten auf, so dass insbesondere Fluide oder Instrumente in dem Arbeitskanal besonders gut transportiert oder bewegt werden können. Zudem kann das Material hohen Temperaturen ausgesetzt werden, beispielsweise bei einer Reinigung des Arbeitskanals in einem Autoklav. Das expandierte PTFE wiederum weist gegenüber dem PTFE verbesserte Festigkeitseigenschaften auf. Es eignet sich dazu, erwärmt und komprimiert zu werden, um die Dicke der weiteren Mantelschicht zu variieren. Die Kombination der Mantelschichten aus PTFE und ePTFE schafft einen besonders flexiblen und gleichzeitig stabilen Arbeitskanal, dessen Durchmesser bzw. Manteldicke nach Bedarf variiert und an die Konstruktion des Instruments angepasst werden kann.

Es wird zudem vorgeschlagen, dass das Kunststoffmaterial der Mantelschicht frei von Poren ausgebildet ist. Es kann vorteilhaft eine Fluiddichtigkeit des Arbeitskanalmantels verbessert werden. Bevorzugt weist das Kunststoffmaterial der Mantelschicht eine Dichte von weniger als 3 g/cm3auf. Dies ist insbesondere bei PTFE gegeben. Die Dichte des Kunststoffmaterials der Mantelschicht ist insbesondere entlang der Haupterstreckung des Arbeitskanals konstant.

Es wird ferner vorgeschlagen, dass die weitere Mantelschicht ein weiteres Kunststoffmaterial aufweist, welches Poren aufweist. Es kann vorteilhaft eine Flexibilität und gleichzeitig eine Stabilität des Arbeitskanalmantels verbessert werden.

Ferner wird vorgeschlagen, dass die weitere Mantelschicht ein weiteres Kunststoffmaterial mit Poren aufweist und im Bereich einer abnehmenden Schichtdicke frei von Poren ausgebildet ist. Bei Verwendung von ePTFE als weiteres Kunststoffmaterial weist dieses Poren auf, kann im Bereich einer verringerten oder abnehmenden Schichtdicke jedoch frei von Poren sein, beispielsweise wenn es dort erhitzt und komprimiert wurde, um die Schichtdicke zu variieren. Dies kann insbesondere wie zuvor beschrieben in wenigstens einem weiteren Abschnitt der Fall sein. Dieser Abschnitt mit einer äußeren Oberfläche ohne Poren eignet sich dann besonders gut, um mit anderen Bauteilen der Endoskopvorrichtung oder des Endoskops verklebt oder anderweitig stoffschlüssig verbunden zu werden.

Bevorzugt weist das Kunststoffmaterial der weiteren Mantelschicht eine Dichte von weniger als 3 g/cm3, insbesondere weniger als 2 g/cm³ auf). Das Kunststoffmaterial weist insbesondere eine Dichte von höchstens 1,7 g/cm3 und besonders bevorzugt von höchstens1,6 g/cm3. Ferner kann das Kunststoffmaterial der weiteren Mantelschicht insbesondere eine Dichte von wenigstens 0,6 g/cm3 vorzugsweise wenigstens 0,8 g/cm3 und besonders bevorzugt von wenigstens 1,0 g/cm3 auf. Das Kunststoffmaterial weist insbesondere abschnittsweise entlang einer Haupterstreckung des Arbeitskanals eine variable Dichte auf. Die Dichte des Kunststoffmaterials der Mantelschicht variiert insbesondere entlang der Haupterstreckung des Arbeitskanals konstant. Vorzugsweise ist eine Variation der Dichte des Kunststoffmaterials an die Variation der Schichtdicke der weiteren Mantelschicht gekoppelt. Die Dichte kann insbesondere im Bereich des weiteren Abschnitts vergrößert sein, beispielsweise wenn das weitere Kunststoffmaterial komprimiert wurde um die Dicke der weiteren Mantelschicht zu verringern.

Die Dichte des Kunststoffmaterials der weiteren Mantelschicht ist insbesondere geringer, als die des Kunststoffmaterials der einen Mantelschicht.

Zudem wird vorgeschlagen, dass die Endoskopvorrichtung wenigstens eine distale Endoskopspitze umfasst mit welcher der Arbeitskanal stoffschlüssig und/oder formschlüssig verbunden ist. Der Arbeitskanal kann insbesondere in einem distalen Abschnitt mit der Endoskopspitze verklebt sein. Auf diese Weise wird der Arbeitskanal in seiner Lage relativ zum Endoskop fixiert und bleibt trotzdem flexibel.

In einem weiteren Aspekt der Erfindung wird ein Endoskop mit wenigstens einer solchen Endoskopvorrichtung vorgeschlagen. Hierdurch kann eine Herstellung und eine Standzeit des Endoskops verbessert werden. Insbesondere kann das Endoskop wenigstens zwei oder mehrere solcher Endoskopvorrichtungen umfassen.

In einem weiteren Aspekt der Erfindung wird ein Verfahren zur Herstellung einer wie zuvor beschrieben ausgebildeten Endoskopvorrichtung für ein flexibles Endoskop vorgeschlagen, und ist durch die Merkmale des Anspruchs 17 definiert.

### Zeichnungen

Weitere Vorteile ergeben sich aus der folgenden Zeichnungsbeschreibung. In den Zeichnungen sind zwei Ausführungsbeispiele dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
Fig. 1 eine offene Darstellung einer Endoskopvorrichtung
Fig. 2 einen Ausschnitt eines Arbeitskanals einer Endoskopvorrichtung
Fig. 3 einen Ausschnitt eines alternativen Arbeitskanals einer Endoskopvorrichtung
Fig. 4 ein Verfahren zur Herstellung einer Endoskopvorrichtung

### Beschreibung der Ausführungsbeispiele

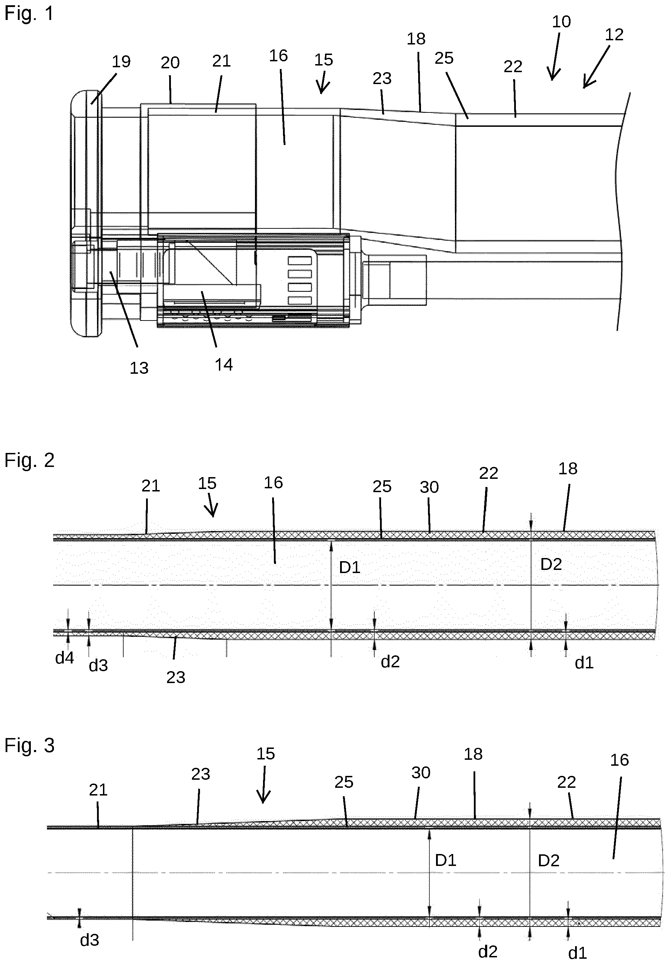

Fig. 1 zeigt eine schematische, offene Darstellung einer Endoskopvorrichtung 10 eines Endoskops in einer seitlichen Ansicht des distalen Endbereichs der Vorrichtung. Das Endoskop ist im vorliegenden Fall als ein flexibles Endoskop ausgebildet. Alternativ könnte das Endoskop jedoch auch als ein starres Endoskop ausgebildet sein. Teile des Endoskopschafts 12 sind in der Figur nicht dargestellt, um eine Sicht auf die innenliegenden Bauteile zu ermöglichen.

Die Endoskopvorrichtung 10 bildet im vorliegenden Fall einen Teil des Endoskops. Sie könnte das Endoskop auch vollständig ausbilden. Die Endoskopvorrichtung 10 weist einen Endoskopschaft 12 auf. Der Endoskopschaft 12 ist flexibel ausgebildet. In dem Endoskopschaft 12 sind weitere Komponenten der Endoskopvorrichtung 10 angeordnet, wie beispielsweise ein Irrigationskanal, Lichtwellenleiter oder dergleichen. Diese sind der Übersichtlichkeit halber nicht dargestellt. In der Figur sind innerhalb eines distalen Bereichs des Endoskopschafts 12 (links in der Figur) eine Optik 13 und ein Bildsensor 14 dargestellt.

Die Endoskopvorrichtung 10 weist einen Arbeitskanal 15 auf. Der Arbeitskanal 15 ist in dem Endoskopschaft 12 angeordnet. Der Arbeitskanal 15 weist ein Arbeitskanalvolumen 16 auf. Der Arbeitskanal 15 weist eine Arbeitskanalmantel 18 auf. Die Arbeitskanalmantel 18 schließt das Arbeitskanalvolumen 16 ein. Der Arbeitskanalmantel 18 weist entlang einer Haupterstreckung des Arbeitskanals einen konstanten Innendurchmesser auf. Derart ist in dem Arbeitskanalvolumen 16 in einem Betriebszustand wenigstens ein chirurgisches Instrument anordenbar. Der Arbeitskanal 15 verläuft zudem leicht abgewinkelt von proximal (rechts in der Figur) nach distal.

Die Endoskopvorrichtung 10 weist zur Anordnung der Komponenten der Endoskopvorrichtung 10 eine distale Endoskopspitze 19 auf. Dazu weist die Endoskopspitze 19 eine Arbeitskanalaufnahme 20 auf, in welcher ein distaler Endabschnitt des Arbeitskanals 15 anordenbar ist. Der Arbeitskanal 15 ist stoffschlüssig mit der Endoskopspitze 19 verbunden, insbesondere verklebt. Alternativ könnte der Arbeitskanal 15 jedoch auch kraft- und/oder formschlüssig mit der Endoskopspitze 19 verbunden sein.

Zu einer Anordnung des Arbeitskanals 15 an der Endoskopspitze 19 weist der Arbeitskanalmantel 18 entlang der Haupterstreckung des Arbeitskanals 15 einen variablen Außendurchmesser auf. Der Arbeitskanalmantel 18 weist einen distalen Abschnitt 21 auf. Im vorliegenden Fall verjüngt sich der Arbeitskanalmantel 18 abschnittsweise in distaler Richtung. Der Außendurchmesser nimmt in distaler Richtung abschnittsweise ab.

Der Arbeitskanalmantel 18 weist einen proximalen Abschnitt 22 auf. In dem proximalen Abschnitt 22 ist der Außendurchmesser des Arbeitskanalmantels 18 entlang der Haupterstreckung des Arbeitskanals 15 konstant. Ferner weist der Arbeitskanalmantel 18 einen distalen Abschnitt 21 auf. Der distale Abschnitt 21 ist im vorliegenden Fall ein distaler Endabschnitt des Arbeitskanals 15. In dem proximalen Abschnitt 22 ist der Außendurchmesser entlang der Haupterstreckung des Arbeitskanal 15 konstant. Eine Wanddicke des Arbeitskanalmantels 18 ist in dem proximalen Abschnitt 22 entlang der Haupterstreckung des Arbeitskanals konstant. Der Außendurchmesser des Arbeitskanalmantels 18 ist im distalen Abschnitt ist 21 wenigstens um 10% kleiner als der Außendurchmesser im proximalen Abschnitt 22. Eine Wanddicke des Arbeitskanalmantels 18 ist in dem distalen Abschnitt 21 entlang der Haupterstreckung des Arbeitskanals konstant. Die Wanddicke in dem distalen Abschnitt ist wenigstens um 10 % kleiner als die Wanddicke im proximalen Abschnitt 22. Ferner weist der Arbeitskanalmantel 18einen Mittelabschnitt 23 auf. Der Mittelabschnitt 23 ist zwischen dem distalen Abschnitt 21 und dem proximalen Abschnitt 22 angeordnet. In dem Mittelabschnitt 23 nimmt der Außendurchmesser des Arbeitskanalmantels 18 kontinuierlich entlang der Haupterstreckung des Arbeitskanals 15 ab. In dem Mittelabschnitt 23 geht der Außendurchmesser des proximalen Abschnitts 22 kontinuierlich in den Außendurchmesser des distalen Abschnitts 21 über. In dem Mittelabschnitt 23 nimmt die Wanddicke des Arbeitskanalmantels 18 kontinuierlich entlang der Haupterstreckung des Arbeitskanals 15 ab. In dem Mittelabschnitt 23 geht die Wanddicke des proximalen Abschnitts 22 kontinuierlich in die Wanddicke des distalen Abschnitts 21 über.

Die Details des Arbeitskanalmantels 18 sind in einer Ausführungsform in Figur 2 dargestellt. Die Figur zeigt einen distalen Ausschnitt des Arbeitskanals 15, wie er zur Figur 1 beschrieben wurde, wobei der Kanal 15 hier ohne Abwinkelung, mit gleichbleibendem Innendurchmesser von proximal nach distal verläuft (in der Figur von rechts nach links). Der Arbeitskanalmantel 18 weist wenigstens eine Mantelschicht 25 auf. Die Mantelschicht 25 bildet eine Innenseite des Arbeitskanalmantels 18 aus. Die Mantelschicht 25 umfasst wenigstens ein Kunststoffmaterial. Im vorliegenden Fall umfasst die Mantelschicht 25 nur ein Kunststoffmaterial. In anderen Worten bildet das Kunststoffmaterial die Mantelschicht 25 vollständig aus. Bei dem Kunststoffmaterial handelt es sich im vorliegenden Fall um Polytetrafluorethylen (PTFE). Alternativ könnte der Kunststoffmaterial auch ein anderer thermisch stabiler Kunststoff sein, welcher einem Autoklavierprozess stand hält. Das Kunststoffmaterial der Mantelschicht 25 ist frei von Poren ausgebildet. Das Kunststoffmaterial hat eine Dichte von weniger als 3 g/cm3 auf. Ferner weist das Kunststoffmaterial eine Dichte von höchstens 2,4 g/cm3. Im vorliegenden Fall weist das Kunststoffmaterial eine Dichte von 2,2 g/m3 auf.

In den Figuren 2 und 3 sind die Durchmesser und verschiedenen Dicken durch Pfeile angedeutet. Es bezeichnen D1 den Innendurchmesser des Arbeitskanalmantels 18, D2 den Außendurchmesser des Kanalmantels 18, d1 die Dicke der weiteren Mantelschicht 30 in einem proximalen Bereich, d2 die Dicke des Mantels 18 in einem proximalen Bereich 22, d3 die Dicke der weiteren Mantelschicht 30 in einem distalen Bereich 21 und d4 die Dicke der Mantelschicht 25. Die weiteren Bezugszeichen bezeichnen die gleichen Teile wie bereits zu Figur 1 ausgeführt.

Die Mantelschicht 25 weist eine Schichtdicke d4 auf. Die Schichtdicke d4 der Mantelschicht 25 entspricht wenigstens 2% des Innendurchmessers D1 des Arbeitskanalmantels 18. Die Schichtdicke d4 ist entlang der Haupterstreckung des Arbeitskanals 15 konstant. Die Haupterstreckungsrichtung ist in der Figur 3 als gestrichelte Linie längs des Arbeitskanals 15 dargestellt. Die Schichtdicke d4 der Mantelschicht weist einen gleichen Wert in den verschiedenen Abschnitten 21, 22 und 23 des Arbeitskanals 15 auf.

Der Arbeitskanalmantel 18 weist wenigstens eine weitere Mantelschicht 30 auf. Die weitere Mantelschicht 30 bildet eine Außenseite des Arbeitskanalmantels 18 aus. Die Mantelschicht 25 und die weitere Mantelschicht 30 liegen aneinander an. Die Mantelschicht 25 und die weitere Mantelschicht 30 sind stoffschlüssig miteinander verbunden. Alternativ oder zusätzlich könnten die Mantelschicht 25 und die weitere Mantelschicht 30 auch formschlüssig miteinander verbunden sein.

Die weitere Mantelschicht 30 umfasst wenigstens ein weiteres Kunststoffmaterial. Im vorliegenden Fall umfasst die weitere Mantelschicht 30 nur ein Kunststoffmaterial. In anderen Worten bildet das weitere Kunststoffmaterial die weitere Mantelschicht 30 vollständig aus.

Das weitere Kunststoffmaterial ist von dem Kunststoffmaterial der Mantelschicht 25 verschieden ausgebildet. Bei dem weiteren Kunststoffmaterial handelt es sich im vorliegenden Fall um expandiertes Polytetrafluorethylen (ePTFE). Alternativ könnte der Kunststoffmaterial auch ein anderer thermisch stabiler expandierter Kunststoff sein, welcher einem Autoklavierprozess stand hält. Das weitere Kunststoffmaterial der weiteren Mantelschicht 30 weist aufgrund des Expansionsprozesses bei seiner Herstellung Poren auf. Das weitere Kunststoffmaterial weist eine Dichte von höchstens 1,9 g/cm3 auf. Ferner hat das Kunststoffmaterial eine Dichte von wenigstens 0,6 g/cm3. Im vorliegenden Fall weist das Kunststoffmaterial eine Dichte von 1,6 g/cm3 auf, wobei die Dichte in einem Abschnitt mit geringerer Dicke d3 der Mantelschicht 30 höher sein kann, beispielsweise wenn das Material komprimiert wurde.

Die weitere Mantelschicht 30 weist eine weitere Schichtdicke d1, d3 auf. Die weitere Schichtdicke d1, d3 ist entlang der Haupterstreckung des Arbeitskanals 15 variabel. Die Schichtdicke d1, d3 der weiteren Mantelschicht 30 nimmt abschnittsweise entlang der Haupterstreckung des Arbeitskanals 15 ab. Im vorliegenden Fall nimmt die Schichtdicke d1, d3 der weiteren Mantelschicht abschnittsweise entlang der Haupterstreckung des Arbeitskanals 15 in distaler Richtung ab.

In dem proximalen Abschnitt 22 weist die weitere Mantelschicht 30 eine Schichtdicke d1 auf. Diese Schichtdicke d1 entspricht hier etwa 10% des Innendurchmessers D1 des Arbeitskanals 15. In dem Mittelabschnitt 23 nimmt die Schichtdicke der weiteren Mantelschicht 30 kontinuierlich ab. Im vorliegenden Fall nimmt die Schichtdicke in dem Mittelabschnitt 23 bis auf den Wert d3 ab, der etwa der Hälfte der Dicke d1 entspricht.

Die Wanddicke d2 besteht aus der Summe der Schichtdicke d4 der Mantelschicht 25 und der weiteren Schichtdicke d2 der weiteren Mantelschicht 30.

Figur 3 zeigt eine weitere Ausgestaltung der Endoskopvorrichtung. Gleiche Bezugszeichen bezeichnen auch hier gleiche Teile wie in den Figuren 1 und 2 dargestellt. Die Ausführungsform des Arbeitskanals 15 der Endoskopvorrichtung 10 unterscheidet sich von der in Figur 2 dargestellten dadurch, dass ein distaler Abschnitt 21 des Arbeitskanalmantels 18 frei von der weiteren Mantelschicht 30 ist. Die weiteren Dimensionen und Eigenschaften des Kanalmantels 18 und der Mantelschichten 25 und 30 sind wie zuvor beschrieben. In einem Mittelabschnitt 23 des Mantels 18 verringert sich die Schichtdicke der weiteren Mantelschicht 30 hier jedoch entlang der Haupterstreckung des Kanals 15 von einem Wert d1 ungleich Null im proximalen Bereich 22 auf einen Wert d3 gleich Null oder nahezu Null im distalen Abschnitt 21. Der distale Abschnitt 21 kann also frei von der weiteren Mantelschicht 30 sein und der Mantel 18 ist hier nur durch die Mantelschicht 25 ausgebildet.

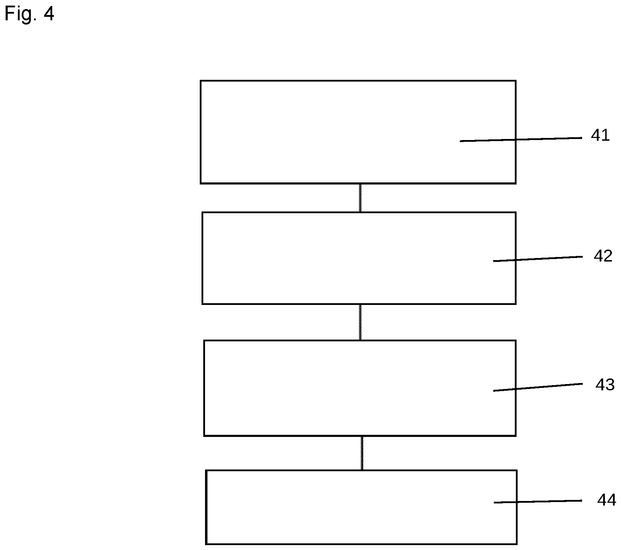

Fig. 4 zeigt einen schematischen Ablaufplan eines beispielhaften Verfahrens zur Herstellung einer Endoskopvorrichtung. Die Endoskopvorrichtung kann wie zuvor beschrieben ausgebildet sein.

Das Verfahren umfasst wenigstens einen Verfahrensschritt 41. In dem Verfahrensschritt 41wird ein Arbeitskanal 15 ausgebildet. Die Mantelschicht 25 des Arbeitskanalmantels 18 wird durch Extrusion des Kunststoffmaterials oder ein anderes Verfahren hergestellt.

Das Verfahren umfasst wenigstens einen weiteren Verfahrensschritt 42. In dem weiteren Verfahrensschritt 42 wird die weitere Mantelschicht 30 des Arbeitskanalmantels 18 hergestellt. Ferner wird die weitere Mantelschicht 30 stoffschlüssig mit der Mantelschicht 25 verbunden. Dazu wird das weitere Kunststoffmaterial auf die Mantelschicht 25 aufgebracht und anschließend durch Wärme expandiert.

Das Verfahren umfasst wenigstens eine weiteren Verfahrensschritt 43. In dem weiteren Verfahrensschritt 43 wird die Dicke der weiteren Mantelschicht 30 entlang der Haupterstreckung des Arbeitskanals 15 wenigstens abschnittsweise variiert. Dazu wird die weitere Mantelschicht 30 in dem Abschnitt erwärmt und mit Druck beaufschlagt. Dadurch wird der weitere Kunststoff der weiteren Mantelschicht 30 verdichtet und dadurch in den Kunststoff der Mantelschicht 25 zumindest teilweise umgewandelt. Dazu kann der Arbeitskanalmantel 18 in einen spitz zulaufenden Trichter eingeführt werden bis dieser in Kontakt mit ihm gelangt. Um die weitere Mantelschicht 30 oder deren Schichtdicke zu variieren, insbesondere zu verringern oder zu entfernen kann beispielsweise ein Drehverfahren oder auch ein Ätzverfahren Anwendung finden.

Das Verfahren umfasst einen weiteren Verfahrensschritt 44. In dem weiteren Verfahrensschritt 44 wird der Arbeitskanalmantel 18 mit der Endoskopspitze 19 stoffschlüssig verbunden. Dazu wird ein Adhäsiv auf den Arbeitskanalmantel 18 appliziert und der Arbeitskanalmantel 18 in die Arbeitskanalaufnahme 20 der Endoskopspitze 19 eingeführt.

Das Verfahren kann weitere Schritte zur Verbesserung des Arbeitskanals und der Endoskopvorrichtung umfassen.

Die Erfindung wird durch die nachstehenden Ansprüche definiert.

## Patentansprüche

1. Endoskopvorrichtung (10) für ein Endoskop mit wenigstens einem in einem Endoskopschaft anordenbaren Arbeitskanal (15), welcher dazu geeignet ist, darin ein chirurgisches Instrument anzuordnen oder um darin Flüssigkeiten oder Gase zu transportieren und einen schlauchförmigen Arbeitskanalmantel (18) aufweist, welcher ein Arbeitskanalvolumen (16) des Arbeitskanals (15), begrenzt, wobei der Arbeitskanalmantel (18) wenigstens eine Mantelschicht (25) aufweist, welche wenigstens ein Kunststoffmaterial umfasst, und wenigstens eine weitere Mantelschicht (30) aufweist, welche wenigstens ein weiteres Kunststoffmaterial umfasst, welches von dem Kunststoffmaterial der einen Mantelschicht (25) verschieden ist, dass die eine Mantelschicht (25) entlang der Haupterstreckung des Arbeitskanals (15) eine konstante Schichtdicke aufweist, und dass die weitere Mantelschicht (30) entlang der Haupterstreckung des Arbeitskanals (15) abschnittsweise eine variable Schichtdicke aufweist, **dadurch gekennzeichnet, dass** das Kunststoffmaterial der weiteren Mantelschicht (30) Poren aufweist und in einem Bereich (23) einer abnehmenden Schichtdicke frei von Poren ausgebildet ist.

2. Endoskopvorrichtung (10) nach Anspruch 1, **dadurch gekennzeichnet, dass** die Schichtdicke der weiteren Mantelschicht (30) abschnittsweise entlang der Haupterstreckung des Arbeitskanals (15) abnimmt.

3. Endoskopvorrichtung (10) nach Anspruch 1 oder 2, **dadurch gekennzeichnet, dass** die Schichtdicke der weiteren Mantelschicht (30) abschnittsweise entlang der Haupterstreckung des Arbeitskanals (15) in distaler Richtung abnimmt.

4. Endoskopvorrichtung (10) nach einem der vorhergehenden Ansprüche, **dadurch gekennzeichnet, dass** der Arbeitskanalmantel (18) wenigstens abschnittsweise frei von der weiteren Mantelschicht (30) ist.

5. Endoskopvorrichtung (10) nach Anspruch 4, **dadurch gekennzeichnet, dass** der Arbeitskanalmantel (18) in einem distalen Endabschnitt (21) frei von der weiteren Mantelschicht (30) ist.

6. Endoskopvorrichtung (10) nach einem der vorhergehenden Ansprüche, **dadurch gekennzeichnet, dass** die Mantelschicht (25) und die weitere Mantelschicht (30) aneinander anliegen.

7. Endoskopvorrichtung (10) nach einem der vorhergehenden Ansprüche, **dadurch gekennzeichnet, dass** die Mantelschicht (25) und die weitere Mantelschicht (30) stoffschlüssig miteinander verbunden sind.

8. Endoskopvorrichtung (10) nach einem der vorhergehenden Ansprüche, **dadurch gekennzeichnet, dass** die Mantelschicht (25) eine Innenseite des Arbeitskanalmantels (18) ausbildet und die weitere Mantelschicht (30) eine Außenseite des Arbeitskanalmantels (18) ausbildet.

9. Endoskopvorrichtung (10) nach einem der vorhergehenden Ansprüche, **dadurch gekennzeichnet, dass** ein Innendurchmesser des Arbeitskanalmantels (18) entlang der Haupterstreckung des Arbeitskanals (15) konstant ist.

10. Endoskopvorrichtung (10) nach einem der vorhergehenden Ansprüche, **dadurch gekennzeichnet, dass** ein Außendurchmesser des Arbeitskanalmantels (18) entlang der Haupterstreckung des Arbeitskanals (15) variabel ist.

11. Endoskopvorrichtung (10) nach einem der vorhergehenden Ansprüche, **dadurch gekennzeichnet, dass** wenigstens eines der Kunststoffmaterialien Polytetrafluorethylen (PTFE) ist.

12. Endoskopvorrichtung (10) nach einem der vorhergehenden Ansprüche, **dadurch gekennzeichnet, dass** das eine Kunststoffmaterial der einen Mantelschicht (25) Polytetrafluorethylen (PTFE) ist und das weitere Kunststoffmaterial der weiteren Mantelschicht (30) expandiertes Polytetrafluorethylen (ePTFE) ist.

13. Endoskopvorrichtung (10) nach einem der vorhergehenden Ansprüche **dadurch gekennzeichnet, dass** das Kunststoffmaterial der weiteren Mantelschicht (30) zumindest im Bereich einer konstanten Schichtdicke eine Dichte von weniger als 3 g/cm³ , insbesondere weniger als 2 g/cm³ aufweist.

14. Endoskopvorrichtung (10) nach einem der vorhergehenden Ansprüche, **dadurch gekennzeichnet, dass** das Kunststoffmaterial der weiteren Mantelschicht (30) zumindest im Bereich einer konstanten Schichtdicke eine geringere Dichte aufweist, als jenes der einen Mantelschicht (25) .

15. Endoskopvorrichtung (10) nach einem der vorhergehenden Ansprüche, **gekennzeichnet durch** wenigstens eine distale Endoskopspitze (19) mit welcher der Arbeitskanal (15) stoffschlüssig verbunden ist.

16. Endoskop mit wenigstens einer Endoskopvorrichtung (10) nach einem der vorhergehenden Ansprüche.

17. Verfahren zur Herstellung einer Endoskopvorrichtung (10) nach einem der Ansprüche 1 bis 16 in wenigstens einem Verfahrensschritt eine konstante Schichtdicke der Mantelschicht entlang der Haupterstreckung des Arbeitskanals (15) konstant gehalten wird und eine Schichtdicke der weiteren Mantelschicht (30) entlang der Haupterstreckung des Arbeitskanals (15) abschnittsweise variiert wird, **dadurch gekennzeichnet, dass** das Kunststoffmaterial der weiteren Mantelschicht (30) Poren aufweist und in einem Bereich (23) einer abnehmenden Schichtdicke frei von Poren ausgebildet ist.

## Claims

1. An endoscope device (10) for an endoscope with at least one working channel (15) which can be arranged in an endoscope shaft, is suitable for arranging a surgical instrument therein or for transporting liquids or gases therein and has a tubular working channel sheath (18), which delimits a working channel volume (16) of the working channel (15),
wherein the working channel sheath (18) has at least one sheath layer (25), which comprises at least one plastic material, and at least one further sheath layer (30), which comprises at least one further plastic material, which is different from the plastic material of the one sheath layer (25), in that the one sheath layer (25) has a constant layer thickness along the main extent of the working channel (15), and in that the further sheath layer (30) has a variable layer thickness in sections along the main extent of the working channel (15), **characterised in that** the plastic material of the further sheath layer (30) has pores and is free of pores in a region (23) of decreasing layer thickness.

2. The endoscope device (10) according to claim 1, **characterised in that** the layer thickness of the further sheath layer (30) decreases in sections along the main extent of the working channel (15).

3. The endoscope device (10) according to claim 1 or 2, **characterised in that** the layer thickness of the further sheath layer (30) decreases in sections along the main extent of the working channel (15) in the distal direction.

4. The endoscope device (10) according to one of the preceding claims, **characterised in that** the working channel sheath (18) is at least in sections free of the further sheath layer (30).

5. The endoscope device (10) according to claim 4, **characterised in that** the working channel sheath (18) is free of the further sheath layer (30) in a distal end section (21).

6. The endoscope device (10) according to one of the preceding claims, **characterised in that** the sheath layer (25) and the further sheath layer (30) lie against each other.

7. The endoscope device (10) according to one of the preceding claims, **characterised in that** the sheath layer (25) and the further sheath layer (30) are connected to one another in a materially-bonded manner.

8. The endoscope device (10) according to one of the preceding claims, **characterised in that** the sheath layer (25) forms an inner side of the working channel sheath (18) and the further sheath layer (30) forms an outer side of the working channel sheath (18).

9. The endoscope device (10) according to one of the preceding claims, **characterised in that** an inner diameter of the working channel sheath (18) is constant along the main extent of the working channel (15).

10. The endoscope device (10) according to one of the preceding claims, **characterised in that** an outer diameter of the working channel sheath (18) is variable along the main extent of the working channel (15).

11. The endoscope device (10) according to one of the preceding claims, **characterised in that** at least one of the plastic materials is polytetrafluoroethylene (PTFE).

12. The endoscope device (10) according to one of the preceding claims, **characterised in that** the one plastic material of the one sheath layer (25) is polytetrafluoroethylene (PTFE) and the other plastic material of the further sheath layer (30) is expanded polytetrafluoroethylene (ePTFE).

13. The endoscope device (10) according to one of the preceding claims, **characterised in that** the plastic material of the further sheath layer (30) has a density of less than 3 g/cm³, in particular less than 2 g/cm³, at least in the region of a constant layer thickness.

14. The endoscope device (10) according to one of the preceding claims, **characterised in that** the plastic material of the further sheath layer (30) has a lower density, at least in the region of a constant layer thickness, than that of the one sheath layer (25).

15. The endoscope device (10) according to one of the preceding claims, **characterised by** at least one distal endoscope tip (19) to which the working channel (15) is connected in a materially-bonded manner.

16. An endoscope with at least one endoscope device (10) according to one of the preceding claims.

17. A method for producing an endoscope device (10) according to one of claims 1 to 16, wherein in at least one method step a constant layer thickness of the sheath layer is kept constant along the main extent of the working channel (15) and a layer thickness of the further sheath layer (30) is varied in sections along the main extent of the working channel (15), **characterised in that** the plastic material of the further sheath layer (30) has pores and is free of pores in a region (23) of decreasing layer thickness.

## Revendications

1. Dispositif d'endoscope (10) pour un endoscope avec au moins un canal de travail (15) qui peut être agencé dans une tige d'endoscope, étant adapté pour y agencer un instrument chirurgical ou pour y transporter des liquides ou des gaz et a une gaine de canal de travail tubulaire (18), qui délimite un volume de canal de travail (16) du canal de travail (15),
dans lequel la gaine de canal de travail (18) a au moins une couche de gaine (25), qui comprend au moins une matière plastique, et au moins une couche de gaine supplémentaire (30), qui comprend au moins une matière plastique supplémentaire, qui est différente de la matière plastique de ladite couche de gaine (25), en ce que la première couche de gaine (25) a une épaisseur de couche constante le long de l'étendue principale du canal de travail (15), et en ce que la couche de gaine supplémentaire (30) a une épaisseur de couche variable par sections le long de l'étendue principale du canal de travail (15), **caractérisé en ce que** la matière plastique de la couche de gaine supplémentaire (30) a des pores et est exempte de pores dans une région (23) d'épaisseur de couche décroissante.

2. Dispositif d'endoscope (10) selon la revendication 1, **caractérisé en ce que** l'épaisseur de couche de la couche de gaine supplémentaire (30) diminue par sections le long de l'étendue principale du canal de travail (15).

3. Dispositif d'endoscope (10) selon la revendication 1 ou 2, **caractérisé en ce que** l'épaisseur de couche de la couche de gaine supplémentaire (30) diminue par sections le long de l'étendue principale du canal de travail (15) dans la direction distale.

4. Dispositif d'endoscope (10) selon l'une des revendications précédentes, **caractérisé en ce que** la gaine de canal de travail (18) est au moins par sections exempte de la couche de gaine supplémentaire (30).

5. Dispositif endoscopique (10) selon la revendication 4, **caractérisé en ce que** la gaine de canal de travail (18) est exempte de la couche de gaine supplémentaire (30) dans une section d'extrémité distale (21).

6. Dispositif d'endoscope (10) selon l'une des revendications précédentes, **caractérisé en ce que** la couche de gaine (25) et la couche de gaine supplémentaire (30) reposent l'une contre l'autre.

7. Dispositif d'endoscope (10) selon l'une des revendications précédentes, **caractérisé en ce que** la couche de gaine (25) et la couche de gaine supplémentaire (30) sont reliées l'une à l'autre d'une manière matériellement liée.

8. Dispositif d'endoscope (10) selon l'une des revendications précédentes, **caractérisé en ce que** la couche de gaine (25) forme un côté interne de la gaine de canal de travail (18) et la couche de gaine supplémentaire (30) forme un côté externe de la gaine de canal de travail (18).

9. Dispositif d'endoscope (10) selon l'une des revendications précédentes, **caractérisé en ce qu'**un diamètre interne de la gaine de canal de travail (18) est constant le long de l'étendue principale du canal de travail (15).

10. Dispositif d'endoscope (10) selon l'une des revendications précédentes, **caractérisé en ce qu'**un diamètre externe de la gaine de canal de travail (18) est variable le long de l'étendue principale du canal de travail (15).

11. Dispositif d'endoscope (10) selon l'une des revendications précédentes, **caractérisé en ce qu'**au moins l'une des matières plastiques est du polytétrafluoroéthylène (PTFE).

12. Dispositif d'endoscope (10) selon l'une des revendications précédentes, **caractérisé en ce que** ladite matière plastique de ladite couche de gaine (25) est du polytétrafluoroéthylène (PTFE) et l'autre matière plastique de la couche de gaine supplémentaire (30) est du polytétrafluoroéthylène expansé (ePTFE).

13. Dispositif d'endoscope (10) selon l'une des revendications précédentes, **caractérisé en ce que** la matière plastique de la couche de gaine supplémentaire (30) a une densité inférieure à 3 g/cm³, en particulier moins de 2 g/cm³, au moins dans la région d'une épaisseur de couche constante.

14. Dispositif d'endoscope (10) selon l'une des revendications précédentes, **caractérisé en ce que** la matière plastique de la couche de gaine supplémentaire (30) a une densité inférieure, au moins dans la région d'une épaisseur de couche constante, que celle de ladite couche de gaine (25).

15. Dispositif d'endoscope (10) selon l'une des revendications précédentes, **caractérisé par** au moins une pointe d'endoscope distale (19) à laquelle le canal de travail (15) est relié de manière matériellement liée.

16. Endoscope avec au moins un dispositif d'endoscope (10) selon l'une des revendications précédentes.

17. Procédé de fabrication d'un dispositif d'endoscope (10) selon l'une des revendications 1 à 16, dans lequel, dans au moins une étape de procédé, une épaisseur de couche constante de la couche de gaine est maintenue constante le long de l'étendue principale du canal de travail (15) et une épaisseur de couche de la couche de gaine supplémentaire (30) varie par sections le long de l'étendue principale du canal de travail (15), **caractérisé en ce que** la matière plastique de la couche de gaine supplémentaire (30) a des pores et est exempte de pores dans une région (23) d'épaisseur de couche décroissante.
